# FASCICULE DE BREVET EUROPEEN

(11) **EP 1 660 640 B1**
(45) Date de publication et mention de la délivrance du brevet: **20.06.2007**
(21) Numéro de dépôt: 04787309.6
(22) Date de dépôt: 03.09.2004
(51) Int. Cl.: C12N 1/38, A23C 9/12, A23C 19/032, A23C 9/13

(54) **ACTIVATEUR POUR FERMENT A BASE DE BACTERIES LACTIQUES ET PROCEDE DE PREPARATION D UN PRODUIT METTANT EN OEUVRE LEDIT ACTIVATEUR**
AUF MILCHSÄUREBAKTERIEN BERUHENDER FERMENTAKTIVATOR UND VERFAHREN ZUR HERSTELLUNG EINES PRODUKTS UNTER VERWENDUNG DES AKTIVATORS
FERMENT ACTIVATOR BASED ON LACTIC ACID BACTERIA, AND METHOD OF PREPARING A PRODUCT USING SAID ACTIVATOR

(30) Priorité: 03.09.2003 FR 0310423
(43) Date de publication de la demande: 31.05.2006
(73) Titulaire: DANISCO A/S, 1411 Copenhagen K. (DK)
(72) Inventeur: BERGER, Claudette, F-91540 Mennecy (FR); HUPPERT, Sonia, F-94300 Vincennes (FR); MORNET, Annie, F-86230 Mondion (FR)
(74) Mandataire: Nargolwalla, Cyra
(86) Numéro de dépôt international: PCT/FR2004/002254
(87) Numéro de publication internationale: WO 2005/024001

(56) Documents cités:
- EP-A- 0 059 113
- EP-A- 1 201 748
- FR-A- 2 814 469
- STERN N J ET AL: "LACTOBACILLUS ACIDOPHILUS UTILIZATION OF SUGARS AND PRODUCTION OF A FERMENTED SOYBEAN PRODUCT" CANADIAN INSTITUTE FOR FOOD SCIENCE AND TECHNOLOGY. JOURNAL, XX, XX, vol. 10, no. 3, 1977, pages 197-200, XP001015436 ISSN: 0315-5463
- DATABASE BIOSIS [Online] BIOSCIENCES INFORMATION SERVICE, PHILADELPHIA, PA, US; 1999, GOUESBET G ET AL: "Lactobacillus delbrueckii subsp. bulgaricus and heat stress" XP002285581 Database accession no. PREV200000179640 & MEDEDELINGEN FACULTEIT LANDBOUWKUNDIGE EN TOEGEPASTE BIOLOGISCHE WETENSCHAPPEN UNIVERSITEIT GENT, vol. 64, no. 5a, 1999, pages 259-265,

## Description

La présente invention se rapporte à un activateur pour un ferment à base de bactéries lactiques, à l'utilisation de cet activateur pour la préparation de produits industriels ou alimentaires et au procédé de préparation de ce produit caractérisé par la mise en oeuvre de cet activateur.

Les bactéries lactiques sont utilisées dans de nombreuses industries, notamment dans l'industrie agro-alimentaire. Elles sont utilisées entres autres pour fermenter, aromatiser, affiner ou texturer les aliments, notamment les produits laitiers ou les produits de charcuterie. Elles sont également utilisées pour protéger les milieux dans lesquels elles sont incorporées contre les contaminations par d'autres micro-organismes et également utilisées pour leur effet probiotique.

Selon les applications, les bactéries lactiques sont commercialisées sous forme de compositions comprenant des mélanges de bactéries lactiques, que l'on appelle ferment ou starter.

Les bactéries lactiques les plus utilisées et qui sont présentes dans les ferments, sont ceux appartenant aux genres *Lactococcus, Streptococcus, Lactobacillus, Leuconostoc, Pediococcus, Bifidobacterium, Brevibacterium, Carnobacterium, Enterococcus, Micrococcus, Vagococcus, Staphylococcus, Bacillus, Kocuria, Arthrobacter* et *Corynebacterium.* Ces bactéries lactiques sont utilisées seules ou en mélanges.

On peut également citer parmi les bactéries lactiques, les bactéries de type thermophile notamment les *Streptococcus thermophilus, Lactobacillus casei, Lactobacillus paracasei, Lactobacillus helveticus, Lactobacillus delbrueckii subsp. Bulgaricus, Lactobacillus bulgaricus* et *Lactobacillus acidophilus,* cette liste n'est pas exhaustive.

Ces ferments se présentent généralement sous la forme de concentrés soit sous forme sèche, lyophilisée, congelée soit sous forme de suspension et sont utilisés le plus souvent sous forme de suspension. Dans le cas des bactéries lactiques sous forme sèche, lyophilisée ou congelée, leurs utilisations nécessitent une mise en suspension préalable.

Ces types de formulation concentrées ont pour double avantage de préserver la viabilité des cultures sur une large période de temps et d'être tout particulièrement appropriés à l'ensemencement direct, selon lequel on introduit directement le ferment dans le milieu à traiter ou à ensemencer. Avantageusement dans ce dernier cas, aucune mise en culture préliminaire dans un milieu de culture ne s'avère nécessaire avant utilisation du ferment par opposition à l'ensemencement dit semi-direct.

Bien que la présente invention puisse également être appliquée efficacement à l'ensemencement semi-direct, elle s'avère tout particulièrement intéressante pour l'ensemencement dit direct pour la raison suivante : lorsque les bactéries sont introduites dans le milieu à traiter ou à ensemencer, par exemple le lait de fabrication, lors d'un ensemencement direct, c'est-à-dire sous la forme d'un concentrât sec, liquide ou congelé, elles ne sont pas immédiatement efficaces et elles nécessitent un temps de remise en activité. La remise en activité de ce type de ferment nécessite un laps de temps d'adaptation correspondant d'une part au rétablissement de la bactérie conditionnée sous sa forme naturelle (phase de réhydratation de la bactérie) et d'autre part à la restitution de son activité métabolique.

Les industriels ont donc mis au point des activateurs destinés à être mis en contact avec les ferments préalablement à l'ensemencement direct ou semi-direct de manière à remettre en activité les ferments.

Or dans le cas de la remise en activité des bactéries lactiques, les activateurs disponibles actuellement ne conviennent pas car ils ne permettent pas de conserver l'activité des bactéries lactiques ainsi que leurs propriétés texturantes, notamment pendant des périodes d'au moins 24 heures d'activation et à température ambiante.

EP-A-1 201 748 décrit un activateur pour un ferment à base de bactéries lactiques comprenant au moins un saccharide choisi dans le groupe constitué par les maltodextrines, les maltodextrines branchées, les amidons solubles, les oligosaccharides seuls ou en mélange, permettant une diminution du temps de latence de croissance bactérienne, une acidification plus rapide et une population bactérienne importante en nombre et présentant une viabilité élevée. La stabilité de la population bactérienne au cours du stockage n'est pas évaluée.

FR-A-2 814 469 décrit également un activateur pour ferment à base de bactéries lactiques, comprenant au moins une substance azotée, un système tampon, et au plus 5% en poids en sucre métabolisable par lesdites bactéries lactiques; préférentiellement exempt de sucre, permettant de réduire significativement la période de latence de croissance bactérienne.

Stern et al décrivent dans J. Inst. Can. Sci. Technol. Aliment., Vol. 10, No. 3, 197, pages 197-200, que l'aptitude de huit lignées de *Lactobacillus acidophilus* à utiliser certains saccharides variait avec la lignée.

Afin de répondre aux exigences des industriels il est devenu nécessaire de trouver un activateur pour bactéries lactiques dont les propriétés permettent de maintenir les propriétés des bactéries lactiques, notamment à température ambiante.

De manière inattendue, les inventeurs ont mis en évidence que la mise en contact d'un ferment à base de bactéries lactiques et avec un activateur conforme à l'invention, préalablement à son introduction dans le milieu à traiter ou ensemencer, permettait de conserver la stabilité de l'activité de ces bactéries.

Par l'expression « conserver la stabilité de l'activité de ces bactéries », on entend que les bactéries conservent leurs propriétés d'acidification du milieu à traiter ou à ensemencer alors qu'elles sont réactivées par l'activateur et ne sont pas encore ensemencées dans ledit milieu à traiter ou à ensemencer, sans qu'il y ait de multiplication cellulaire ou très peu.

Par l'expression « milieu à traiter ou à ensemencer » on entend le milieu dans lequel est introduit le ferment, activé ou non. Il peut s'agir par exemple d'un milieu à base de lait, ou de jus de fruit ou d'un extrait de soja.

Dans ce but la présente invention propose pour premier objet un activateur pour un ferment à base de bactéries lactiques, caractérisé en ce qu'il comprend au moins :
- un disaccharide réducteur,
- un disaccharide non réducteur,
- un sel de métal alcalin et / ou un sel de métal alcalino-terreux.

Elle a pour second objet l'utilisation de cet activateur pour activer un ferment à base de bactéries lactiques, préalablement à ou lors de l'ensemencement direct dans un milieu à traiter.

Un autre aspect de la présente invention concerne un ferment à base de bactéries lactiques activées par l'activateur selon l'invention.

Enfin, la présente invention a pour quatrième objet un procédé de préparation d'un produit contenant au moins un ferment caractérisé par la mise en oeuvre de cet activateur ou d'un ferment activé selon l'invention.

La technique de l'ensemencement direct offre des avantages déterminants : disponibilité immédiate des ferments sous un encombrement réduit, possibilité de réaliser des mélanges complexes d'espèces ou de souches différentes dans des proportions déterminées et constantes, régularité accrue des performances par rapport aux ferments traditionnels préparés sur les lieux d'utilisation, production réalisée dans des unités spécialisées où chaque étape du procédé est optimisée et contrôlée, qualité des ferments rigoureusement définie.

L'activateur selon l'invention est particulièrement intéressant en terme de stabilité d'un ferment à ensemencement direct sous forme liquide.

Avantageusement l'activateur selon l'invention permet de réactiver un ferment dans un liquide aqueux, et notamment dans de l'eau.

En conséquence, l'utilisation conjointe de l'activateur avec un ferment à base de bactéries lactiques permet avantageusement de préserver et standardiser l'activité métabolique des bactéries activées, sur une période de temps prolongée comparativement à celle observée avec un même ferment sous une forme non activée.

De plus, de manière tout à fait avantageuse, l'utilisation de l'activateur avec un ferment permet de retarder la multiplication cellulaire ou tout simplement de limiter la multiplication cellulaire, tout en permettant aux ferments de reprendre leur activité métabolique et en maintenant efficace le ferment activé selon l'invention.

L'activateur selon l'invention convient tout particulièrement à des ferments contenant entre autres des micro-organismes dits thermophiles ayant une température de croissance optimale entre 35 et 45°C, mais pouvant s'étendre à des températures comprises entre 30 et 50°C.

Enfin l'activateur selon l'invention a pour avantage de pouvoir être mis en oeuvre dans toutes industries, notamment l'industrie agro-alimentaire, pharmaceutique, cosmétique, alimentaire, l'agriculture, ainsi que dans les domaines de la nutrition animale, des aliments pour animaux et de l'hygiène au sens large en particulier l'hygiène corporelle (par exemple les dentifrices) ou l'hygiène industrielle.

D'autres avantages et caractéristiques de l'invention apparaîtront clairement à la lecture de la description et des exemples donnés à titre purement illustratifs et non limitatifs qui vont suivre.

L'invention concerne tout d'abord un activateur pour un ferment à base de bactéries lactiques, caractérisé en ce qu'il comprend au moins :
- un disaccharide réducteur,
- un disaccharide non réducteur,
- un sel de métal alcalin et / ou un sel de métal alcalino-terreux.

Cet activateur convient de préférence pour les ferments à base de bactéries lactiques thermophiles.

L'activateur selon l'invention contient au moins un disaccharide réducteur. Parmi les disaccharides réducteurs qui conviennent selon l'invention, on peut citer le lactose, le lactulose, le maltose, le cellobiose ou l'allolactose.

Le disaccharide réducteur peut être ajouté dans l'activateur sous forme de composé pur ou sous forme d'un mélange impur comme c'est le cas par exemple du lait en poudre ou du lactosérum de fromagerie ou de caséinerie, qui contiennent au moins un disaccharide réducteur.

L'activateur selon l'invention contient également au moins un disaccharide non réducteur.

Parmi les disaccharides non réducteurs qui conviennent selon l'invention, on peut citer le saccharose, le thréalose ou le raffinose.

L'activateur selon l'invention contient aussi au moins un sel de métal alcalin et / ou un sel de métal alcalino-terreux. De préférence il s'agit d'un sel de sodium, de potassium, de calcium ou de magnésium comme par exemple le chlorure de sodium, de calcium, de magnésium ou de potassium, le phosphate de sodium ou de potassium, l'orthophosphate de sodium ou de potassium, le citrate de sodium ou de potassium, ou le formiate de sodium ou de potassium.

Les proportions relatives de chaque constituants compris dans l'activateur sont les suivantes :
- 30 à 50 % de disaccharides réducteurs,
- 30 à 50 % de disaccharides non réducteurs,
- 10 à 30 % de sels de métal alcalin et / ou un sel de métal alcalino-terreux, les pourcentages étant exprimés en poids.

Un ferment activé avec l'activateur selon l'invention est de manière avantageuse, efficace sur une période s'étendant jusqu'à 72 heures, plus particulièrement sur une période s'étendant jusqu'à 48 heures, préférentiellement sur une période s'étendant jusqu'à 24 heures.

C'est ainsi qu'un ferment à base de bactéries activées selon l'invention est efficace sur une période s'étendant jusqu'à 72 heures alors qu'un même ferment réhydraté dans l'eau et non activé, manifeste une perte d'activité significative au-delà de trois heures.

Par ailleurs, les inventeurs ont constaté que la présence de l'activateur était avantageuse en terme d'équilibre des populations microbiennes du système activé.

Un gain de productivité particulièrement significatif peut être obtenu pour des ferments à base de bactéries thermophiles.

Comme il ressort des exemples figurant ci-après, un ferment à base de bactéries lactiques activé selon l'invention préalablemant à son introduction dans le milieu à traiter restitue beaucoup plus rapidement un pouvoir acidifiant dans le milieu à traiter comparativement au ferment standart, c'est à dire sous forme non-activée.

Selon une variante de l'invention, sont également associés à l'activateur selon l'invention, des éléments nutritifs nécessaires au maintien de l'activité métabolique des bactéries lactiques.

Ces éléments nutritifs incluent généralement des vitamines, des extraits de levure, des acides aminés, des peptides ou des protéines.

De même, peuvent être présents dans l'activateur selon l'invention des co-facteurs utiles pour activer la glycolyse. A titre représentatif de ces co-facteurs, on peut en particulier citer les sels minéraux Ca²⁺, e, Mg²⁺, Mn²⁺, Cu²⁺ et Zn²⁺. Ils sont généralement utilisés à raison de 0,1 à 2% en poids.

On peut également envisager d'incorporer dans l'activateur des agents de texture, comme par exemples des polysaccharides ou des hydrocolloïdes, en particulier des carraghénanes ou des gommes xanthane, de guar, de caroube, de tara.

L'activateur selon l'invention peut être obtenu par simple mélange de ses composants et se présente généralement sous une forme sèche, généralement pulvérulente. Toutefois, on peut également envisager de le formuler sous une forme lyophilisée ou congelée.

L'activateur selon l'invention peut aussi se présenter sous forme liquide.

Selon une variante préférée de l'invention, l'activateur selon l'invention se présente sous une forme stérilisée et est mis en oeuvre en respectant cet aspect stérile.

La présente invention a pour second objet l'utilisation d'un activateur conforme à la présente invention pour activer un ferment à base de bactéries lactiques préalablement à ou lors de l'ensemencement direct dans un milieu à traiter ou à ensemencer.

De préférence la mise en contact dudit activateur avec le ferment à base de bactéries lactiques est réalisée en milieu liquide, en particulier de l'eau.

L'utilisation de cet activateur pour activer en milieu liquide un ferment à base de bactéries lactiques permet un ensemencement en ligne continu ou discontinu, automatisable, et aseptique.

L'invention a également pour objet un ferment à base de bactéries lactiques activées, caractérisé en ce qu'il associe à des bactéries lactiques, un activateur conforme à l'invention.

En l'espèce, l'activateur selon l'invention est utilisé en quantité telle que ces composants sont présents en quantités suffisantes pour que l'on observe une activation significative du ferment à base de bactéries lactiques.

Le ratio, masse de ferment sur masse d'activateur est compris entre 0,1 et 0,7, de préférence entre 0,2 et 0,6.

Le ferment à base de bactéries lactiques activées selon l'invention peut être préparés de manière à ce que les bactéries lactiques et l'activateur sont associés au sein d'un milieu liquide, en particulier de l'eau.

L'activateur peut être mélangé au ferment soit préalablement ou au moment de son utilisation. Toutefois, selon un mode de réalisation privilégié, on procède préalablement à l'utilisation du ferment, à sa réhydratation en présence d'un activateur conforme à la présente invention. Généralement, cette association est réalisée dans un milieu liquide, de préférence l'eau.

L'activateur est réhydraté de manière que la quantité d'activateur soit comprise entre 5 % et 20 % en poids de suspension aqueuse, de préférence comprise entre 7 % et 15%.

La réhydratation et l'activation consécutive du ferment peuvent être réalisées à une température ambiante, notamment à une température comprise entre 15°C et 25°C, de préférence comprise entre 18°C et 23°C et plus particulièrement sous agitation, de manière à optimiser l'activation et l'homogénéisation dans le temps. Le ferment activé est ensuite utilisé tel quel pour l'ensemencement, de préférence direct, d'un milieu à traiter.

Les bactéries lactiques susceptibles d'être associées à un activateur conforme à l'invention incluent toutes les bactéries lactiques usuellement mises en oeuvre dans l'industrie notamment l'industrie agro-alimentaire, pharmaceutique, cosmétique, alimentaire, l'agriculture, ainsi que dans les domaines de la nutrition animale, des aliments pour animaux et de l'hygiène au sens large en particulier l'hygiène corporelle (par exemple les dentifrices) ou l'hygiène industrielle.

L'activateur selon l'invention convient également pour les bactéries lactiques thermophiles.

A titre indicatif de bactéries lactiques, on peut citer les bactéries appartenant aux genres *Streptococcus, Lactococcus, Lactobacillus, Leuconostoc, Bifidobacterium* et Pediococcus et notamment *Lactococcus lactis, Lactococcus lactis subsp. diacetylactis, Lactococcus cremoris, Leuconostoc mesenteroides.*

On considère également comme bactéries lactiques thermophiles, les bactéries utilisées dans le domaine laitier appartenant aux genres Propionibacterium, *Brevibacterium* et *Bifidobacterium,* comme par exemple *Bifidobacterium lactis, Bifidobacterium bifidum, Bifidobacterium longum, Bifidobacterium infantis* ou *Bifidobacterium adolescentis.*

La présente invention a pour quatrième objet un procédé de préparation d'un produit contenant au moins un ferment comprenant les étapes suivantes:
(i) la mise en contact d'un ferment comprenant au moins des bactéries lactiques avec un activateur conforme à la présente invention, de manière à obtenir un ferment sous une forme activée,
(ii) l'ensemencement du milieu à traiter, avec ledit ferment sous une forme activée.

Pour ce qui est de l'étape préliminaire (i), à savoir la mise en contact du ferment avec l'activateur revendiqué, elle est généralement effectuée dans un délai suffisant à l'obtention de la forme activée et au sein d'un milieu liquide, en particulier de l'eau. La suspension correspondante peut être obtenue par ajout d'un liquide, de préférence un milieu aqueux, au mélange des deux composants (activateur et ferment) ou par dispersion consécutive des deux composants dans ledit liquide.

Le procédé selon l'invention peut comprendre en outre une étape (iii) d'incubation dudit milieu à traiter dans des conditions favorables à l'activité métabolique des bactéries lactiques, de manière à obtenir un produit fermenté.

La mise en oeuvre du procédé selon l'invention peut se faire grâce à un dispositif d'ensemencement.

Le dispositif d'ensemencement préféré, pour mettre en oeuvre le procédé selon l'invention, peut se présenter sous la forme d'un réservoir scellé.

Le dispositif d'ensemencement, pour mettre en oeuvre le procédé selon l'invention, peut aussi se présenter sous la forme d'un réservoir jetable et / ou fixé sur un poste mobile

Le réservoir scellé peut se présenter sous la forme d'une poche munie d'un système d'agitation interne et de moyens d'entrée et de sortie.

Un des moyens d'entrée permet l'arrivée du milieu aqueux dans le réservoir scellé afin de réaliser l'étape (i). Le milieu aqueux est préalablement stérilisé, de préférence il est filtré sur membrane d'au plus 0,45 µm, plus particulièrement au plus 0,22 µm. Il est à noter que l'on peut utiliser l'eau du robinet.

Un des autres moyens d'entrée permet l'arrivée de gaz dans le réservoir scellé. L'arrivée de gaz va permettre de mettre en oeuvre le système d'agitation interne du réceptacle.

Dans un cas particulier de l'invention, le système d'agitation interne peut être constitué d'une poche interne perméable. Dans ce cas le réservoir scellé comprend une poche interne perméable et une poche externe fermée. L'agitation est réalisée par injection successive de gaz dans la poche interne perméable, qui permet le transfert de la suspension de la poche interne perméable à la poche externe fermée.

Selon un autre cas, le système d'agitation est constitué par la forme en U du réservoir scellé. Dans ce cas l'agitation est réalisée par injection successive de gaz dans un bras du U, qui permet le transfert de la suspension d'un coté à l'autre du U.

On utilise avantageusement un gaz qui peut être de l'air ou un gaz qui n'intervient pas dans la respiration et / ou l'oxydation des micro-organismes, des ferments et des bactéries, ou un gaz chimiquement et biologiquement inerte, par exemple de l'argon, de l'azote ou du dioxyde de carbone ou leurs mélanges.

Par gaz biologiquement inerte, on entend un gaz qui n'intervient pas dans la multiplication et la dégradation des micro-organismes.

La pression de gaz dans le réservoir scellé, au cours de l'agitation, est inférieure à 5 bars, de préférence inférieure à 1 bar.

L'injection de gaz peut également se faire par intervalle régulier de temps. De préférence on injecte le gaz sous pression par intervalle de temps compris entre 0,5 minutes et 60 minutes.

L'agitation permet la mise en suspension des ferments et de l'activateur dans le milieu aqueux.

Après agitation, la suspension de ferments et de l'activateur est maintenue en suspension par injection de gaz selon le même principe d'injection successive de gaz.

La vidange du réservoir scellé se fait de manière aseptique par le moyen de sortie, ce qui permet de réaliser l'étape (ii) du procédé.

Cette vidange est réalisée par injection de gaz à l'intérieur du réservoir scellé, ou par transfert de la suspension aqueuse de ferments et d'activateur à l'aide d'une pompe ou par gravité.

L'ensemencement du milieu à traiter avec ledit ferment sous une forme activée (étape (ii)) est réalisé à un débit compris entre 10 ml/min et 1000 ml/min, de préférence compris entre 100 mi/min et 500 ml/min.

La mise en oeuvre de l'étape (ii) selon l'invention se fait à une température comprise entre 5°C et 45°C.

La mise en oeuvre de l'étape (ii) selon l'invention se fait sur une période s'étendant jusqu'à 72 heures, plus particulièrement sur une période s'étendant jusqu'à 48 heures, préférentiellement sur une période s'étendant jusqu'à 24 heures.

L'étape (ii) peut se réaliser selon plusieurs variantes.

Une première variante du procédé au niveau de l'étape (ii) consiste à ensemencer le milieu à traiter en une seule fois avec ledit ferment sous une forme activée. Ceci est réalisé par vidange du ou des réservoir(s) en une seule fois. Il s'agit d'un ensemencement en lot (un seul réservoir) ou multi-lots (plusieurs réservoirs).

Une seconde variante du procédé au niveau de l'étape (ii) consiste à ensemencer le milieu à traiter de manière continue avec ledit ferment sous une forme activée.

Une troisième variante du procédé au niveau de l'étape (ii) consiste à ensemencer le milieu à traiter de manière discontinue avec ledit ferment sous une forme activée.

Par discontinue on entend un cycle d'ensemencement réalisé de la manière suivante : on ensemence le milieu à traiter pendant un laps de temps, puis on arrête l'ensemencement, puis on recommence l'ensemencement, ceci pendant plusieurs cycles.

Dans le cadre de cette troisième variante, l'ensemencement du milieu à traiter avec ledit ferment sous une forme activée (étape (ii)) est réalisé à un débit compris entre 10 ml/min et 1000 ml/min, de préférence compris entre 100 ml/min et 500 ml/min, fait par intervalle régulier ou irrégulier de temps compris entre 1 minute et 600 minutes.

Il est à noter que le réservoir scellé est de manière avantageuse fixé sur un poste mobile qui peut-être déplacé sur toutes les parties de la chaîne industrielle, avant ou après l'étape (i) du procédé selon l'invention.

Le type de réservoir préféré pour la mise en oeuvre du procédé selon l'invention est du type jetable et / ou stérile.

Ce réservoir est constitué de préférence d'une matière flexible comme par exemple le polypropylène, le polyester, le polyamide, la cellulose ou de tout autre matériel flexible compatible avec les produits alimentaires, de préférence il est en polyéthylène.

L'avantage de la mise en oeuvre du procédé selon l'invention grâce au dispositif d'ensemencement tel que décrit ci-dessus, est de réaliser un ensemencement direct sous forme liquide maintenue à température ambiante, stérile, standardisé, adaptable à chaque type de production et qui garantit la qualité bactériologique.

Un autre avantage de la mise en oeuvre du procédé selon l'invention grâce au dispositif d'ensemencement tel que décrit ci-dessus est de simplifier et fiabiliser l'étape d'ensemencement du ferment lactique.

La présente invention s'étend également aux différentes formes de conditionnement de l'activateur revendiqué.

On peut en effet formuler l'activateur selon l'invention sous un conditionnement distinct de celui du ferment à base de bactéries lactiques auquel il est destiné à être associé ou, à l'inverse, envisager un conditionnement commun au sein duquel sont présents, de manière séparée ou non, l'activateur selon l'invention et le ferment à base de bactéries lactiques.

Cette seconde variante de conditionnement peut par ailleurs être conçue de manière à ce qu'elle soit adaptée au mélange préalable du ferment et de l'activateur et donc à la préparation du ferment dit activé préliminairement à l'ensemencement d'un milieu à traiter.

Les exemples figurant ci-après sont présentés à titre illustratif et non limitatif de la présente invention.

### EXEMPLES

### Méthodes :

Les bactéries lactiques, seules ou en mélange, présentent une grande diversité de comportements. Dans le cas de la présente invention, l'activité acidifiante a été retenue à titre de critère de caractérisation de l'activité des bactéries.

L'acidification d'un milieu lacté a été réalisée selon l'ordre chronologique suivant:
- inoculation d'un lait (pH voisin de 6,6),
- accroissement de la population de bactéries lactiques grâce à l'hydrolyse du lactose du lait,
- production d'acide lactique par les bactéries lactiques qui se traduit par une diminution du pH du milieu lacté,
- arrêt de la croissance des bactéries lactiques qui sont inhibées progressivement par l'acide lactique formé,
- poursuite de la production d'acide jusqu'à un pH de 4,5.

L'activité acidifiante a été appréciée dans les exemples ci-après à l'aide d'un système automatique de suivi et de caractérisation des ferments lactiques par acquisition de mesure de pH en temps réel, encore désigné ci-après sous l'appellation CINAC.

Le CINAC est composé:
* d'électrodes combinées en verre de type Ingold (24 voies de mesures de pH placées dans des erlenmeyers contenant le milieu ensemencé et 8 voies de mesures de température)
* d'un bain-marie régulé par un thermostat et dans lequel sont placés les erlenmeyers
* d'une carte électronique fournissant un signal analogique et une interface électronique convertissant ce dernier en numérique
* d'un micro-ordinateur PC muni du logiciel CINAC assurant les fonctions suivantes :
   - configuration du système
   - acquisitions, traitements et stockages des données
   - étalonnage des sondes à pH7 et pH4
   - calcul des descripteurs cinétiques
   - représentations graphiques des données traitées
   - conversions des données pour l'utilisation de ces dernières sur d'autres logiciels
   - programmation de cycles thermiques pour réguler la température du bain-marie
   - compensation des températures pour corriger les variations de ces dernières sur le pH
      (cette correction se fait grâce à un régulateur PID : proportionnel-intégral-dérivé)
   - exécution de procédures de test des données d'étalonnage afin de détecter les dysfonctionnements liés aux sondes.

Le. CINAC traite les données en fournissant les courbes des cinétiques d'acidification et les descripteurs de ces dernières.

Les courbes, décrivant les cinétiques, représentent les évolutions du pH et de la vitesse d'acidification (dpH/dt), en fonction du temps. Elles témoignent des différentes étapes de la croissance : phase de réadaptation, accélération, phase exponentielle, ralentissement, phase stationnaire.

Les descripteurs retenus dans les exemples pour caractériser les cinétiques d'acidification sont :
* Ta = temps de latence en min (temps au bout duquel le pH a varié de 0.08 upH en dessous de sa valeur initiale)
* Vm = vitesse maximale d'acidification en upH/min (vitesse prise au maximum de la valeur absolue de la dérivée dpH/dt=f(t))
* temps 5,20 = temps pour obtenir un pH de 5,20 en minutes
* temps 4,75 = temps pour obtenir un pH de 4,75 en minutes.

A partir de l'ensemble de ces paramètres il est possible d'apprécier un gain ou une perte de productivité.

Les bactéries bactéries lactiques présentes dans le milieu de réhydratation on été dénombrées au cours du temps selon la méthode suivante :

Le ferment a été réhydraté et activé à l'aide de l'activateur (composition A ou B ci-après), comme indiqué en 1-3. Le ferment activé ainsi obtenu est stocké pendant 24 heures. Au cours de ce stockage, la population bactérienne est mesurée à différents temps de stockage. Cette population est mesurée à différents temps pouvant aller de 1 heure (T1h) à 72 heures (T72h) de stockage.

Les dilutions sont faites dans du tryptone-sel préparé selon le protocole suivant : 1g de tryptone, 8,5g NaCl sont mis dans 1 litre d'eau. La solution obtenue est répartie en tube de 9ml, qui sont alors traités pendant 15 minutes à 120°C.

Les dilutions réalisées à partir de ces tubes sont les suivantes : 10^{E-6} ; 10^{E-7}; 10^{E-8}; 10^{E-9}; 10^{E-10}.

1 ml de ces dilutions est ensuite prélévé et déposé dans les boites de pétri. Les boites sont alors coulées avec différentes géloses puis incubées selon le protocole suivant :

| **Bactéries recherchées** | **Milieu utilisé** | **Inoculation et Incubation** |
|---|---|---|
| *Streptoccocus thermophilus* | M17 Merck prêt à l'emploi | Inoculation en masse |
| | | 37°C - 48h anaérobiose (CO2) |
| *Lactobacillus delbrueckii bulgaricus* | MRS MERCK prêt à l'emploi Acidifié à pH 5,4 | Inoculation en masse |
| | | 37°C - 48h anaérobiose (CO2) |
| *L acidophilus (associé à d'autres bactéréries)* | MRS+ clindamycine à 0,1 mg/l | Inoculation en masse |
| | | 37°C - 48h anaérobiose (CO2) |
| *Bifidobacterium* | MRS + dicloxacilline | Inoculation en masse |
| | | 42°C / 48h / anaérobiose (CO2) |
| *Lactobacillus paracasei paracasei* | MRS MERCK prêt à l'emploi | Inoculation en masse |
| | | 37°C - 48h anaérobiose (CO2) |
| *Lactobacillus acidophilus (utilisé seul)* | MRS MERCK prêt à l'emploi | Inoculation en masse |
| | | 37°C - 48h anaérobiose (CO2) |

### EXEMPLE 1 : Préparation d'activateurs selon l'invention

### 1-1/ Préparation d'un activateur (composition A) :

On prépare l'activateur selon l'invention dans un flacon stérile de 1 I contenant un barreau magnétique double anneaux de 45 mm. Les différents composants de ce mélange sont présentés dans le tableau I ci-après :

**TABLEAU I : Composition A**

| Produits | Quantité (g) |
|---|---|
| Poudre de lait écrémé | 65 |
| Saccharose | 24,5 |
| MgSO4 | 1,6 |
| MgCO3 | 0,8 |
| CaCO3 | 0,8 |
| Extrait de levure | 4,1 |
| MnSO4 | 1,6 |
| Fer citrate ammoniacal | 1,6 |

### 1-2/ Préparation d'un activateur (composition B) :

On prépare l'activateur selon l'invention dans un flacon stérile de 1 contenant un barreau magnétique double anneaux de 45 mm. Les différents composants de ce mélange sont présentés dans le tableau I ci-après :

**TABLEAU II :Composition B**

| Produits | Quantité (g) |
|---|---|
| Lactose | 42 |
| Saccharose | 42 |
| Formiate de sodium | 16 |

### 1-3/ Préparation d'un ferment concentré réhydraté selon l'invention

Dans les exemples ci-après, l'activateur décrit en 1-1 ou 1-2 est ensuite mélangé à 50g de ferment lyophilisé et 870g d'eau stérile. Le mélange sec est versé dans de l'eau sous agitation magnétique et la dissolution se fait en quelques minutes. On obtient ainsi 1 litre d'une solution qui contient 50g de ferment lyophilisé.

La température de réhydratation du mélange résultant, à savoir ferment et activateur est conduite selon un cycle thermique dit « hiver ». Ce cycle restitue la remontée en température d'un ensemble de 25 I qui commence à 15°C et s'achève à une température de 20°C qui est atteinte en 20 heures environ.

### EXEMPLE 2 : Mesure de l'activité acidifiante de différents ferments

### 2-1/ Souches Streptococcus thermophilus

La souche testée est une souche thermophile. Il s'agit plus précisément d'une souches de *Streptococcus thermophilus* qui est un ferment lactique commercialisé par RHODIA FOOD S.A.S.

La souche de *Streptococcus thermophilus* est réhydratée et activée à l'aide de l'activateur (composition A), comme indiqué en 1-3. La souche activée ainsi obtenue est stockée pendant 24 heures à la température indiquée en 1-3. Au cours de ce stockage, l'activité du concentré bactérien est mesurée à différents temps de stockage à l'aide du CINAC comme indiqué précédemment. Cette activité est mesurée après 20 minutes (considéré comme le temps T0), 1 heures (T1h), 3 heures (T3h), 6 heures (T6h), 12 heures (T12h), 16 heures (T16h) et 24 heures (T24h) de stockage.

La souche réhydratée et activée est prélevée aux différents temps de stockage et est ensemencée dans du lait ½ écrémé à 38°C. En raison de la concentration des bactéries, on procède à une dilution pour pouvoir ensemencer les tests d'acidification (1 g de souche activée est dissoute dans 200 ml de lait qui est utilisé pour la mesure d'activité). L'ensemencement doit être réalisé immédiatement pour ne pas pénaliser l'activité du concentrât bactérien.

Une activité témoin est lancée pour chaque test réalisé qui met en oeuvre 1 g de souche lyophilisée dans 200 ml de lait. Les témoins sont des ensemencements directs dans le lait de fabrication avec la souche non activée par l'activateur.

### Mesure de l'activité acidifiante au cours du temps :

Les résultats obtenus avec cette souche sont présentés en tableaux III ci-après. Les données figurant dans ces tableaux rendent compte des gains obtenus en termes de stabilité avec les ferments activés selon l'invention comparativement à leur forme non activée respective.

**TABLEAU III**

| ***Streptococcus thermophilus*** | Ta en min | V max en upH/min | Temps 5,20 | **Population du ferment réhydraté (en unité formant colonie (ufc))** |
|---|---|---|---|---|
| Témoin réhydraté à 20°C | 116 | -0,0157 | 252 | |
| T0 | 105 | -0,0205 | 210 | - |
| T1h | 109 | -0,0215 | 210 | 610.⁹ |
| T3h | 107 | -0,0213 | 210 | |
| T6h | 110 | -0,0215 | 214 | 7 10.⁹ |
| T12h | 109 | -0,0216 | 216 | 6 10.⁹ |
| T16h | 107 | -0,0211 | 213 | 7 10.⁹ |
| T24h | 114 | -0,0216 | 221 | 6 10.⁹ |

Ces résultats indiquent que le temps de latence (Ta) varie très peu, quelques soit le temps de stockage de la souche activée.

De plus au bout de 24 heures de stockage, on constate que la souche de *Streptococcus thermophilus* présente un temps 5,20 de 221 minutes, qui est quasiment identique au temps 5,20 du test T0 (210 minutes). Cela signifie que son activée acidifiante n'est pas altérée au bout de 24 heures de stockage. On note un gain d'activité acidifiante de 42 minutes entre le temps 5,20 du témoin réhydraté (252 minutes) et le test T0 (210 minutes).

De ces résultats il ressort le comportement avantageux de l'activateur vis-à-vis de la population bactérienne présente dans le ferment, et notamment la stabilité de la population bactérienne au cours du stockage.

### 2-2/ Ferment composé de Streptococcus thermophilus et Lactobacillus delbrueckii bulgaricus

Le ferment testé comprend 2 souches de bactéries lactiques, qui sont *Streptococcus thermophilus* et *Lactobacillus delbrueckii bulgaricus.* Il s'agit d'un ferment commercialisé par RHODIA FOOD S.A.S.

Le ferment testé a été réhydraté et activé à l'aide de l'activateur de composition A, selon la méthode indiquée en 1-3. Le ferment activé ainsi obtenu est stocké pendant 24 heures à la température indiquée en 1-3. Au cours de ce stockage, l'activité du concentré bactérien est mesurée à différents temps de stockage à l'aide du CINAC comme indiqué précédemment. Cette activité est mesurée après 1 heures (T1h), 4heures (T4h) et 24 heures (T24h) de stockage.

Les souches réhydratées et activées sont prélevées aux différents temps de stockage et sont ensemencées dans du lait ½ écrémé à 43°C. En raison de la concentration des bactéries, on procède à une dilution pour pouvoir ensemencer les tests d'acidification (1 g de souche activée est dissoute dans 200 ml de lait qui est utilisé pour la mesure d'activité). L'ensemencement doit être réalisé immédiatement pour ne pas pénaliser l'activité du concentrât bactérien.

Une activité témoin est lancée pour chaque test réalisé qui met en oeuvre 1 g de souche lyophilisée dans 200 ml de lait. Les témoins sont des ensemencements directs dans le lait de fabrication avec la souche non activée par l'activateur.

### Mesure de l'activité acidifiante au cours du temps:

Les résultats obtenus avec chacune des souches sont présentés dans le tableau IV ci-après. Les données figurant dans ces tableaux rendent compte des gains obtenus en termes de stabilité et de productivité avec les ferments activés selon l'invention comparativement à leur forme non activée respective.

**TABLEAU IV (Composition A)**

| Ferment pour le yaourt | Ta en min | V max en upH/min | Temps 4,75 | Population totale du ferment réhydraté (en unité formant colonie (ufc)) |
|---|---|---|---|---|
| Témoin réhydraté à 20°C | 85 | - 0,019 | 290 | - |
| T1h | 75 | - 0,019 | 250 | 2.10^{E}10 |
| T4h | 75 | - 0,019 | 250 | 2.10^{E}10 |
| T24h | 85 | - 0,019 | 265 | 2.10^{E}10 |

Les résultats montrent un gain d'activité de 40 minutes pour le temps 4,75 entre le témoin réhydraté (290 minutes) et le ferment activé T1h (250 minutes). Un gain d'activité est observé jusqu'à 24 heures de stockage : le temps 4,75 est plus court. La population totale et l'activité acidifiante est stable durant 24 heures à température comme indiqué au point 1-3.

### 2-3/ Ferment composé de 4 souches

Le ferment testé comprend 4 souches de bactéries lactiques, qui sont *Streptococcus thermophilus, Lactobacillus delbrueckii bulgaricus, Lactobacillus acidophilus* et *bifidobacterium lactis.* Il s'agit d'un ferment commercialisé par RHODIA FOOD S.A.S.

Le ferment a été réhydraté et activé à l'aide de l'activateur (composition B), comme indiqué en 1-3. Le ferment activé ainsi obtenu est stocké pendant 24 heures à la température indiquée en 1-3. Au cours de ce stockage, l'activité du concentré bactérien est mesurée à différents temps de stockage à l'aide du CINAC comme indiqué précédemment. Cette activité est mesurée après 1 heures (T1h), 2 heures (T2h), 4 heures (T4h), 8 heures (T8h), 12 heures (T12h) et 24 heures (T24h) de stockage.

Les souches réhydratées et activées sont prélevées aux différents temps de stockage et sont ensemencées dans du lait ½ écrémé à 43°C. En raison de la concentration des bactéries, on procède à une dilution pour pouvoir ensemencer les tests d'acidification (1 g de souche activée est dissoute dans 200 ml de lait qui est utilisé pour la mesure d'activité). L'ensemencement doit être réalisé immédiatement pour ne pas pénaliser l'activité du concentrât bactérien.

Une activité témoin est lancée pour chaque test réalisé qui met en oeuvre 1 g de souche lyophilisée dans 200 ml de lait. Les témoins sont des ensemencements directs dans le lait de fabrication avec la souche non activée par l'activateur.

### Mesure de l'activité acidifiante au cours du temps :

Les résultats obtenus avec chaque ferment sont présentés dans le tableau V ci-après. Les données figurant dans ce tableau rendent compte des gains obtenus en termes de stabilité et de productivité avec les ferments activés selon l'invention comparativement à leur forme non activée respective.

**TABLEAU V (Composition B)**

| **Ferment pour laits fermentés** | Ta (en min) | V max (en upH/min) | Temps 4,75 | **Population du ferment réhydraté (en unité formant colonies (ufc))** | | |
|---|---|---|---|---|---|---|
| | | | | *S. thermophilus* / *L. bulgaricus* | *L. acidophilus* | *Bifidobacterium* |
| **Témoin réhydraté à 20°C** | **78** | **-0,018** | **300** | - | - | - |
| T1h | 82 | - 0,019 | 275 | 5.10^{E}9 | 3.10^{E}8 | 2.10^{E}8 |
| T2h | 81 | - 0,016 | 290 | 6.10^{E}9 | - | - |
| T4h | 77 | -0,016 | 290 | 5.10^{E}9 | - | - |
| T8h | 80 | -0,016 | 290 | 6.10^{E}9 | 3.10^{E}8 | 2.10^{E}8 |
| T12h | 78 | - 0,017 | 290 | 5.10^{E}9 | 3.10^{E}8 | 2.10^{E}8 |
| T24h | 78 | - 0,016 | 290 | 6.10^{E}9 | 3.10^{E}8 | 2.10^{E}8 |

Les résultats montrent un gain d'activité de 25 minutes pour le temps 4,75 entre le témoin réhydraté (300 minutes) et le ferment activé T1h (275 minutes). Un gain d'activité est observé jusqu'à 24 heures de stockage : le temps 4,75 est plus court. La population totale et l'activité acidifiante est stable durant 24 heures à température comme indiqué au point 1-3.

### EXEMPLE 3 :

### 3-1/ Préparation d'un lait fermenté (yaourts).

Des laits-fermentés (yaourts) sont préparés en utilisant les ferments activés préparés selon l'exemple 2-2. Puis la viscosité du lait fermenté ainsi obtenu est mesurée.

**Préparation des laits fermentés (yaourts) :** Le support de fermentation est obtenu en supplémentant 100 ml de lait UHT ½ écrémé (Petit Vendéen) par 3% (poids/volume) de poudre de lait écrémé (Eurial). La stérilité de la solution est obtenue par une pasteurisation de 10 min à 90°C (à coeur). Le support de fermentation ainsi obtenu est inoculé avec la souche ou le ferment à tester à raison de 4 unités au 100 litres, puis incubé à 43°C (au bain-marie) jusqu'à l'obtention d'un pH de 4,6. Le suivi du pH est réalisé en continu grâce à l'utilisation d'un CINAC (Isbaert). Les yaourts ainsi obtenus sont placés dans une étuve ventilée à 6°C, jusqu'à leur analyse.

**Analyses rhéologiques sur les yaourts :** seule la viscosité est mesurée. Les mesures de viscosité sont réalisées sur des laits fermentés, après 1 et/ou 7 et/ou 14 jours de stockage, dont la température est maintenue à 6°C. L'appareillage utilisé est un viscosimètre Brookfield de type RVF (Brookfield Engineering Laboratories Inc.) monté sur pied Helipath (Brookfield Engineering Laboratories Inc.). Le viscosimètre est équipé d'une aiguille de type C et la vitesse d'oscillation appliquée à l'aiguille est de 10 tours/min

### 3-2/ Résultats de pH et de viscosité sur les yaourts réalisés avec un ferment Streptococcus thermophilus et Lactobacillus delbruekii bulgaricus:

Le ferment testé est identique à celui utilisé au point 2-2 et comprend 2 souches de bactéries lactiques, qui sont *Streptococcus thermophilus* et *Lactobacillus delbrueckii bulgaricus.*

Le ferment testé a été réhydraté et activé à l'aide soit de l'activateur de composition A soit de l'activateur de composition B, selon la méthode indiquée en 1-3. Le ferment activé ainsi obtenu est stocké pendant 24 heures à la température indiquée en 1-3. Au cours de ce stockage, des laits fermentés sont réalisés à différents temps de stockage (1 heure et 24 heures), et la viscosité et le pH sont mesurée comme indiqué précédemment en 3-1.

### Mesure de la viscosité et du pH au cours du temps selon la méthode décrite en 3-1:

Les résultats obtenus avec le ferment sont présentés dans le tableaux VI ci-après. Les données figurant dans ce tableau rendent compte de la stabilité et de la productivité des ferments activés selon l'invention comparativement à leur forme non activée respective.

**TABLEAU VI.**

| Dénomination milieux | | Suivis | | | |
|---|---|---|---|---|---|
| | | J+1 | | J+7 | |
| | | Viscosité en cps | pH | Viscosité en cps | pH |
| Activateur de composition A | T1h | 51 200 | 4.57 | 50 800 | 4.49 |
| | T24h | 50 900 | 4.55 | 49 700 | 4.50 |
| Témoin | T1h | 49 900 | 4.58 | 49 000 | 4.46 |
| | T24h | 50100 | 4.52 | 50100 | 4.43 |

Il n'y a pas de différences significatives entre les yaourts fabriqués avec le témoin et ceux fabriqués avec le ferment activé aussi bien pour le pH que pour la viscosité.

### 3-3/ Résultats de pH et de viscosité sur les yaourts réalisés avec un ferment composé de 4 souches :

Le ferment testé est identique à celui utilisé au point 2-3 et comprend 4 souches de bactéries lactiques, qui sont *treptococcus thermophilus, Lactobacillus delbrueckii bulgaricus, Lactobacillus acidophilus* et *bifidobacterium lactis.*

Le ferment testé a été réhydraté et activé à l'aide de l'activateur de composition B, selon la méthode indiquée en 1-3. Le ferment activé ainsi obtenu est stocké pendant 24 heures à la température indiquée en 1-3. Au cours de ce stockage, des laits fermentés sont réalisés à différents temps de stockage (1 heure, 4 heures, 8 heures et 12 heures), et la viscosité et le pH sont mesurée comme indiqué précédemment en 3-1.

### Mesure de la viscosité et du pH au cours du temps selon la méthode décrite en 3-1 :

Les résultats obtenus avec le ferment sont présentés dans le tableaux VII ci-après. Les données figurant dans ce tableaux rendent compte de la stabilité et de la productivité des ferments activés selon l'invention comparativement à leur forme non activée respective.

**TABLEAU VII:**

| Temps en heures | J+1 | | J+14 | |
|---|---|---|---|---|
| | Viscosité en cps | pH | Viscosité en cps | pH |
| 1 | 33 900 | 4.75 | 39 500 | 4.38 |
| 4 | 33650 | 4.75 | 39050 | 4.39 |
| 8 | 35 100 | 4.64 | 39 000 | 4.34 |
| 12 | 32 400 | 4.70 | 39 500 | 4.44 |
| Temoin | 33000 | 4.74 | 39500 | 4.40 |

Les « yaourts » ou laits fermentés réalisés à partir du ferment réhydraté ont des propriétés similaires à celle du témoin après un stockage de 12h à température comme indiqué en 1-3.

### EXEMPLE 4 : Dénombrement de 2 souches probiotiques

### 4-1/ Souches probiotiques.

Les souches testées sont des souches probiotiques. Il s'agit plus précisément des souches de *Lactobacillus paracasei* (LC) et *Lactobacillus acidophillus* (LA) qui sont des ferments lactiques commercialisés par RHODIA FOOD S.A.S.

Les souches LC et LA sont réhydratées et activées à l'aide de l'activateur (composition B), à un taux de 4,8 10^{E}9 ufc par ml de milieu de réhydratation. Les solutions contenant le milieu de réhydratation et chacune des souches sont réparties en flacons de 125 ml. Ils sont placés dans une pièce dont la température est régulée à 18°C, et mis sous agitation à 150 tours/minutes pendant 72 heures. Au cours de ce stockage, la population en bactéries est déterminée à différents temps de stockage. Ce dénombrement est réalisé après 20 minutes (considéré comme le temps T0), 1 heures (T1h), 5 heures (T5h), 24 heures (T24h), 48 heures (T48h), et 72 heures (T72h) de stockage.

Les résultats obtenus avec chaque ferment sont présentés dans le tableau VIII ci-après.

**TABLEAU VIII :**

| Souches | | dénombrements | | | | |
|---|---|---|---|---|---|---|
| | T0 | T1h | T5h | T24H | T48h | T72h |
| LC | 4,8.10E9 | 5,8.10E9 | 4,6.10E9 | 5,5.10E9 | 4,2.10E9 | 4,8.10E9 |
| LA | 4,8.10E9 | 4,7.10E9 | 3,9.10E9 | 4,2.10E9 | 4,6.10E9 | 3,9.10E9 |

Ces deux souches probiotiques, réhydratées et activées conformément à l'invention, présentent une très bonne stabilité pendant 72 heures de stockage à température comme indiqué au point 1-3.

## Revendications

1. Activateur pour un ferment à base de bactéries lactiques, **caractérisé en ce qu'**il comprend au moins :
- un disaccharide réducteur,
- un disaccharide non réducteur,
- un sel de métal alcalin et / ou un sel de métal alcalino-terreux.

2. Activateur selon la revendication 1, **caractérisé en ce que** les bactéries lactiques sont des bactéries lactiques thermophiles.

3. Activateur selon l'une des revendications précédentes, **caractérisé en ce que** le disaccharide réducteur est le lactose, le lactulose, le maltose, le cellobiose ou l'allolactose.

4. Activateur selon l'une des revendications précédentes **caractérisé en ce que** le disaccharide non réducteur est le saccharose, le thréalose ou le raffinose.

5. Activateur selon l'une des revendications précédentes, **caractérisé en ce que** le sel de métal alcalin et / ou le sel de métal alcalino-terreux est un sel de sodium, de potassium, de calcium ou de magnésium comme par exemple le chlorure de sodium, de calcium, de magnésium ou de potassium, le phosphate de sodium ou de potassium, l'orthophosphate de sodium ou de potassium, le citrate de sodium ou de potassium, ou le formiate de sodium ou de potassium.

6. Activateur selon l'une des revendications précédentes, **caractérisé en ce qu'**il comprend:
- 30 à 50 % de disaccharides réducteurs,
- 30 à 50 % de disaccharides non réducteurs,
- 10 à 30 % de sels de métal alcalin et / ou un sel de métal alcalino-terreux, les pourcentages étant exprimés en poids.

7. Activateur selon l'une des revendications précédentes, **caractérisé en ce qu'**il comprend en outre des éléments nutritifs nécessaires au maintien de l'activité métabolique des bactéries lactiques.

8. Utilisation d'un activateur tel que défini dans les revendications 1 à 7 pour activer un ferment à base de bactéries lactiques, préalablement à ou lors de l'ensemencement direct dans un milieu à traiter ou à ensemencer.

9. Utilisation selon la revendication 8, **caractérisée en ce que** la mise en contact dudit activateur avec le ferment à base de bactéries lactiques est réalisée en milieu liquide, en particulier de l'eau.

10. Ferment à base de bactéries lactiques activées, **caractérisé en ce qu'**il associe à des bactéries lactiques, un activateur selon l'une des revendications 1 à 7.

11. Procédé de préparation d'un produit contenant au moins un ferment comprenant les étapes suivantes:
(i) la mise en contact d'un ferment comprenant au moins des bactéries lactiques avec un activateur selon l'une des revendications 1 à 7, de manière à obtenir le ferment sous une forme activée,
(ii) l'ensemencement du milieu à traiter avec ledit ferment sous une forme activée.

12. Procédé selon la revendication 11, **caractérisé en ce que** la mise en contact du ferment à base de bactéries lactiques avec ledit activateur est réalisée au sein d'un milieu liquide, en particulier de l'eau.

13. Procédé selon l'une des revendications 11 ou 12, **caractérisé en ce que** la mise en oeuvre du procédé peut se faire grâce à un dispositif d'ensemencement, en particulier un réservoir scellé.

14. Procédé selon la revendication 13, **caractérisé en ce que** le réservoir scellé peut se présenter sous la forme d'un réservoir jetable et / ou fixé sur un poste mobile.

15. Procédé selon l'une des revendications 13 ou 14, **caractérisé en ce que** le réservoir scellé peut se présenter sous la forme d'une poche munie d'un système d'agitation interne et de moyens d'entrée et de sortie.

16. Procédé selon l'une des revendications 11 à 15, **caractérisé en ce que** la mise en oeuvre de l'étape (ii) se fait à une température comprise entre 5°C et 45°C.

17. Procédé selon l'une des revendications 11 à 16, **caractérisé en ce que** la mise en oeuvre de l'étape (ii) se fait sur une période s'étendant jusqu'à 72 heures, plus particulièrement sur une période s'étendant jusqu'à 48 heures, préférentiellement sur une période s'étendant jusqu'à 24 heures.

## Claims

1. An activator for a lactic bacteria-based ferment, **characterised in that** it comprises at least:
- one reducing disaccharide,
- one non-reducing disaccharide,
- one alkaline metal salt and/or one alkaline earth metal salt.

2. An activator according to claim 1, **characterised in that** the lactic bacteria are thermophilic lactic bacteria.

3. An activator according to any one of the preceding claims, **characterised in that** the reducing disaccharide is lactose, lactulose, maltose, cellobiose or allolactose.

4. An activator according to any one of the preceding claims, **characterised in that** the non-reducing disaccharide is saccharose, threalose or raffinose.

5. An activator according to any one of the preceding claims, **characterised in that** the alkaline metal salt and/or the alkaline earth metal salt is a sodium-, potassium-, calcium- or magnesium salt, such as for example sodium-, calcium-, magnesium- or potassium chloride, sodium- or potassium phosphate, sodium- or potassium orthophosphate, sodium- or potassium citrate, or sodium or potassium formiate.

6. An activator according to any one of the preceding claims, **characterised in that** it comprises:
- 30 to 50% of reducing disaccharides,
- 30 to 50% of non-reducing disaccharides,
- 10 to 30% of alkaline metal salts and/or an alkaline earth metal salt, the percentages being expressed by weight.

7. An activator according to any one of the preceding claims, **characterised in that** it further comprises nutrients needed for maintaining the metabolic activity of the lactic bacteria.

8. Use of an activator such as defined in claims 1 to 7 for activating a lactic bacteria-based ferment, before or during direct seeding in a medium to be treated or seeded.

9. Use according to claim 8, **characterised in that** the contacting of said activator with the lactic bacteria-based ferment is conducted in liquid medium, particularly water.

10. An activator according to any of one of claims 1 to 7, in combination with an activated lactic bacteria-based ferment.

11. A process for the preparation of a product containing at least one ferment, comprising the following steps:
(i) contacting a ferment comprising at least some lactic bacteria with an activator according to any one of claims 1 to 7, in order to obtain the ferment in an activated form,
(ii) seeding the medium to be treated with said ferment in an activated form.

12. A process according to claim 11, **characterised in that** the contacting of the lactic bacteria-based ferment with said activator is conducted within a liquid medium, particularly water.

13. A process according to claim 11 or 12, **characterised in that** the process can be implemented with the aid of a seeding device, in particular a sealed tank.

14. A process according to claim 13 **characterised in that** the sealed tank can be in the form of a disposable tank and/or a tank which is secured to a movable station.

15. A process according to claim 13 or 14, **characterised in that** the sealed tank can be in the form of a pocket equipped with an internal agitation system and intake and outlet means.

16. A process according to any of one of claims 11 to 15, **characterised in that** step (ii) is carried out at a temperature of between 5°C and 45° C.

17. A process according to any of one of claims 11 to 15, **characterised in that** step (ii) is carried out over a period of time of up to 72 hours, more particularly over a period of time of up to 48 hours, preferably over a period of time of up to 24 hours.

## Patentansprüche

1. Aktivator für ein Ferment auf der Grundlage von Milchsäurebakterien, **dadurch gekennzeichnet, daß** er mindestens folgendes umfaßt:
- ein reduzierendes Disaccharid
- ein nicht-reduzierendes Disaccharid
- ein Alkalimetallsalz und/oder ein Erdalkalimetallsalz.

2. Aktivator gemäß Anspruch 1, **dadurch gekennzeichnet, daß** die Milchsäurebakterien thermophile Milchsäurebakterien sind.

3. Aktivator gemäß einem der vorstehenden Ansprüche, **dadurch gekennzeichnet, daß** das reduzierende Disaccharid Lactose, Lactulose, Maltose, Cellobiose oder Allolactose ist.

4. Aktivator gemäß einem der vorstehenden Ansprüche, **dadurch gekennzeichnet, daß** das nicht-reduzierende Disaccharid Saccharose, Trehalose oder Raffinose ist.

5. Aktivator gemäß einem der vorstehenden Ansprüche, **dadurch gekennzeichnet, daß** das Alkalimetallsalz und/oder das Erdalkalimetallsalz ein Natriumsalz, Kaliumsalz, Calciumsalz oder Magnesiumsalz ist, wie beispielsweise Natriumchlorid, Calciumchlorid, Magnesiumchlorid oder Kaliumchlorid, das Phosphat von Natrium oder Kalium, das Orthophosphat von Natrium oder Kalium, das Citrat von Natrium oder Kalium oder das Formiat von Natrium oder Kalium.

6. Aktivator gemäß einem der vorstehenden Ansprüche, **dadurch gekennzeichnet, daß** er folgendes umfaßt:
- 30 bis 50 % reduzierende Disaccharide
- 30 bis 50 % nicht-reduzierende Disaccharide
- 10 bis 30 % Alkalimetallsalze und/oder Erdalkalimetallsalze,
wobei die Prozentsätze in Gewicht ausgedrückt sind.

7. Aktivator gemäß einem der vorstehenden Ansprüche, **dadurch gekennzeichnet, daß** er außerdem notwendige Nährelemente zum Erhalt der Stoffwechselaktivität der Milchsäurebakterien umfaßt.

8. Verwendung eines Aktivators wie definiert gemäß einem der Ansprüche 1 bis 7 zur Aktivierung eines Ferments auf der Grundlage von Milchsäurebakterien, vor oder während des direkten Beimpfens in ein Behandlungsmedium oder Impfmedium.

9. Verwendung gemäß Anspruch 8, **dadurch gekennzeichnet, daß** das Kontaktieren des Aktivators mit dem Ferment auf der Grundlage von Milchsäurebakterien in einem flüssigen Milieu, insbesondere Wasser, realisiert wird.

10. Ferment auf der Grundlage von aktivierten Milchsäurebakterien, **dadurch gekennzeichnet, daß** man mit Milchsäurebakterien einen Aktivator gemäß einem der Ansprüche 1 bis 7 assoziiert.

11. Verfahren zur Herstellung eines Produkts enthaltend mindestens ein Ferment, umfassend die folgenden Schritte:
(i) Kontaktieren eines Ferments umfassend mindestens Milchsäurebakterien mit einem Aktivator gemäß einem der Ansprüche 1 bis 7 auf eine Weise, um das Ferment in aktivierter Form zu erhalten,
(ii) Beimpfen des Behandelungsmediums mit dem Ferment in seiner aktivierten Form.

12. Verfahren gemäß Anspruch 11, **dadurch gekennzeichnet, daß** das Kontaktieren des Aktivators mit dem Ferment auf der Grundlage von Milchsäurebakterien inmitten eines flüssigen Milieus, insbesondere Wasser, realisiert wird.

13. Verfahren gemäß einem der Ansprüche 11 oder 12, **dadurch gekennzeichnet, daß** sich das Durchführen des Verfahrens aufgrund einer Beimpfvorrichtung, insbesondere eines versiegelten Behälters, durchführen läßt.

14. Verfahren gemäß Anspruch 13, **dadurch gekennzeichnet, daß** der versiegelte Behälter sich in Form eines Wegwerfbehälters präsentieren kann und/oder auf einem mobilen Posten fixiert ist.

15. Verfahren gemäß einem der Ansprüche 13 oder 14, **dadurch gekennzeichnet, daß** der versiegelte Behälter sich in Form einer mit einem internen Rührsystem versehenen Tasche und Mitteln für eine Eingabe und Ausgabe präsentieren kann.

16. Verfahren gemäß einem der Ansprüche 11 bis 15, **dadurch gekennzeichnet, daß** der Schritt (ii) bei einer Temperatur zwischen 5 und 45°C durchgeführt wird.

17. Verfahren gemäß einem der Ansprüche 11 bis 16, **dadurch gekennzeichnet, daß** der Schritt (ii) in einer Zeitspanne bis zu 72 Stunden, genauer gesagt einer Zeitspanne bis zu 48 Stunden, vorzugsweise in einer Zeitspanne bis zu 24 Stunden, durchgeführt wird.
